**Europäisches Patentamt**

**European Patent Office**

**Office européen des brevets**

(11) Publication number: **0 155 560**
**B1**

## (12) EUROPEAN PATENT SPECIFICATION

(45) Date of publication of patent specification: **09.01.91**

(51) Int. Cl.⁵: **A 61 J 1/00,** B 65 D 23/00, B 65 D 39/00

(21) Application number: **85102209.5**

(22) Date of filing: **28.02.85**

(54) Drug delivery system.

(30) Priority: **19.03.84 US 590601**

(43) Date of publication of application:
**25.09.85 Bulletin 85/39**

(45) Publication of the grant of the patent:
**09.01.91 Bulletin 91/02**

(84) Designated Contracting States:
**AT BE CH DE FR GB IT LI LU NL SE**

(56) References cited:
**EP-A-0 096 115**
**FR-A-2 509 689**
**FR-A-2 529 531**
**GB-A-2 064 493**

(73) Proprietor: **ABBOTT LABORATORIES**
**14th Street and Sheridan Road North St**
**North Chicago, Illinois 60064 (US)**

(72) Inventor: **Tripp, Edward S.**
**385 Dorset Court**
**Park City Illinois 60085 (US)**
Inventor: **Larkin, Mark E.**
**419 Northgate**
**Lindenhurst Illinois 60046 (US)**

(74) Representative: **Modiano, Guido et al**
**MODIANO, JOSIF, PISANTY & STAUB Modiano**
**& Associati Via Meravigli, 16**
**I-20123 Milano (IT)**

Courier Press, Leamington Spa, England.

EP 0 155 560 B1

## Description

### Background of the Invention

This invention relates to a drug delivery system which both stores and subsequently affords sterile access to the contents of a drug containing vial. More particularly, this invention relates to a drug delivery system which allows sterile access to a packaged drug, either by the utilization of a syringe or direct insertion of the drug containing vial into a fluid supply source in an I.V. administration system.

Medicaments or drugs normally administered in a health care environment, being flowable substances, are typically packaged in vials whose interior is maintained in a sterile condition. The vials themselves are sealed by a sterile stopper which is eventually pierced by a cannula when it is desired to remove the medicament or drug. Several procedures are required in order to get the drug from the vial and into the body of a patient. Each procedure is time consuming for health care personnel and more importantly each procedure presents a risk of jeopardizing the sterility of the vial, the stopper or the medicament.

If the medicament in a particular vial is a powder the procedures which may jeopardize sterility include adding diluent to the vial to dissolve the powder and then subsequently further diluting the concentrated diluent-medicament solution to the desired strength. When the medicament or drug can be administered directly to a patient, a cannula and syringe assembly may be used to make an injection through the skin of the patient or into a Y-site in an I.V. administration set. If the medicament or drug is of such a strength or potency that it must be administered over a prolonged period of time then the medicament or drug is mixed with a large quantity of diluent and placed into a container which becomes a secondary fluid source in a piggyback I.V. administration system. If there is no primary fluid source in an I.V. administration system the medicament and diluent solution may itself be administered intravenously to a patient.

The problem, therefore, experienced by health care and pharmacy personnel with prior art drug delivery systems is two-fold. First, multiple time consuming procedures are required in order to take the drug from its storage condition to a condition in which it can be properly and safely administered to a patient. Second, each procedure between storage and administration presents a new opportunity for jeopardizing drug stopper or vial sterility. The need exists, therefore, to provide a system by which the possibility of contamination of a drug, the vial and the stopper are minimized between storage and administration.

Nowhere in the art is there found a drug delivery system which minimizes the procedures between storage and administration and simultaneously assures sterility of the drug, the stopper and the vial.

U.S. Patent No. 2,977,014 discloses enclosing a vial within a separate container for protection against damage and U.S. Patent No. 3,394,831 discloses the addition of a tear strip to facilitate opening of a protective package surrounding a medicament container. The utilization of a U-shaped stopper to securely lock and unlock the opening in a vial is disclosed in U.S. Patent 2,746,632. Covering the stopper in the vial with a sealing disk to maintain sterility of the stopper is disclosed in U.S. Patent 4,244,478, and formation of a handle or hanger at the base of a container is disclosed in U.S. Patent 3,325,031 to Singier.

Also known from U.S. Patent No. 4,244,478 is a closure unit for a dose unit vial for oral administration of medication, comprising an elastomer stopper which seals the vial and affords a rim covering the lip of the vial, and a metal sealing ring crimped onto the vial, covering the stopper rim but exposing a linear slit valve in the stopper.

However this closure unit cannot be used in a drug delivery system for minimizing procedures between storage and administration while simultaneously assuring sterility of the drug, the stopper, and the vial.

The above-mentioned problems encountered in the prior art are overcome by a storage and delivery system for a flowable substance as defined in claim 1.

### Summary of the Invention

A system is provided for storage and delivery of a drug or medicament for health care or pharmacy personnel. The drug or medicament is sealed within a vial by a removable, pierceable stopper in the vial opening. Positioned over the stopper in the vial opening is a removable, pierceable diaphragm. Circumscribing the outside of the vial is a skirt member which is in frictional contact with the wall portion of the vial. The diaphragm is connected to the skirt member so that access to the interior of the vial may be obtained by either piercing the diaphragm and the stopper with the point of a cannula or completely separating the diaphragm from the skirt member so that the stopper may be removed from the vial opening. In the preferred embodiment the separation of the diaphragm from the skirt member is accomplished by the use of frangible sections circumscribing that portion of the skirt member which connects the portion of the skirt member in frictional contact with the wall portion of the vial and the diaphragm. Once the diaphragm has been removed from its position over the stopper, the stopper may be removed from the vial and the contents of the vial added to an intravenous administration system without compromising sterility of the drug, the vial or the stopper.

### Brief Description of the Drawings

A further understanding of the drug delivery system of this invention may be had by reference to the drawings wherein:

FIGURE 1 is a perspective view of the drug delivery system of this invention.

FIGURE 2 is a side elevational view of the drug delivery system of this invention in conjunction with an I.V. administration system.

FIGURE 3 is a front view in partial section of the drug delivery system illustrated in FIGURE 1.

FIGURE 4 is an exploded assembly view of the drug delivery system illustrated in FIGURE 1.

FIGURE 5 is a sectional view of the drug delivery system of the present invention in use with a syringe.

FIGURE 6 is a perspective view of the drug delivery system of the present invention illustrating removal of the tear cap.

FIGURE 7 is a side elevational view in partial section of the drug delivery system of the present invention inserted in a port on a flexible diluent container.

FIGURE 8 is a side elevational view in partial section of the drug delivery system of the present invention in fluid communication with the diluent in a flexible diluent container.

FIGURE 9 is an exploded assembly view of an alternate embodiment of the drug delivery system of the present invention.

Description of the Embodiments

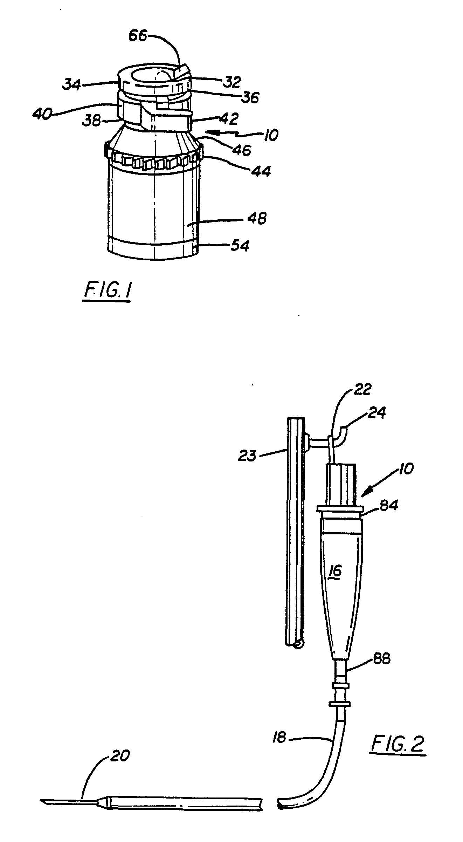

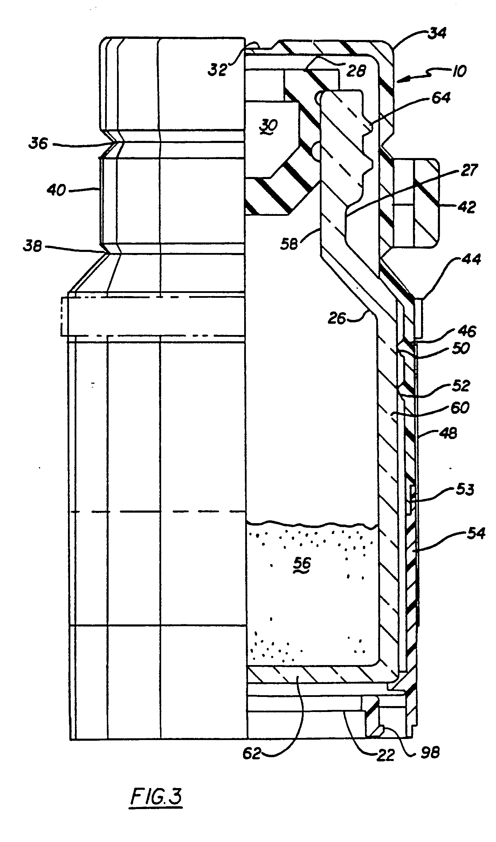

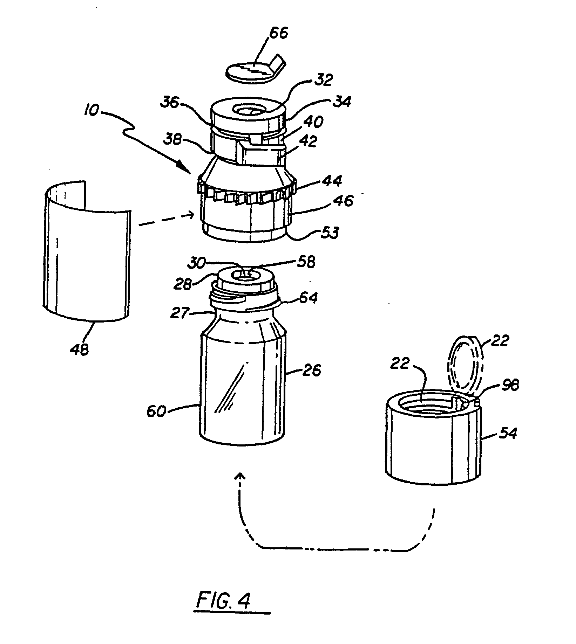

FIGURE 1 illustrates the preferred embodiment of the drug delivery system 10 as it will be received by health care personnel or hospital pharmacies. The drug is identified to the user by label 48. Shroud member 54 and skirt member 46 form a sleeve which surrounds the drug-containing vial 26 (FIGURES 3 and 4). Integral with skirt member 46 is tear strip 40 which may be removed from system 10 by grasping tab 42. Once tear strip 40 has been removed, pierceable diaphragm 32 may also be removed so as to expose the top of vial 26. When in place, however, diaphragm 32, tear strip 40 and skirt member 46 maintain the upper portions of vial 26 and stopper 28 (FIGURES 3 and 4) in a sterile condition.

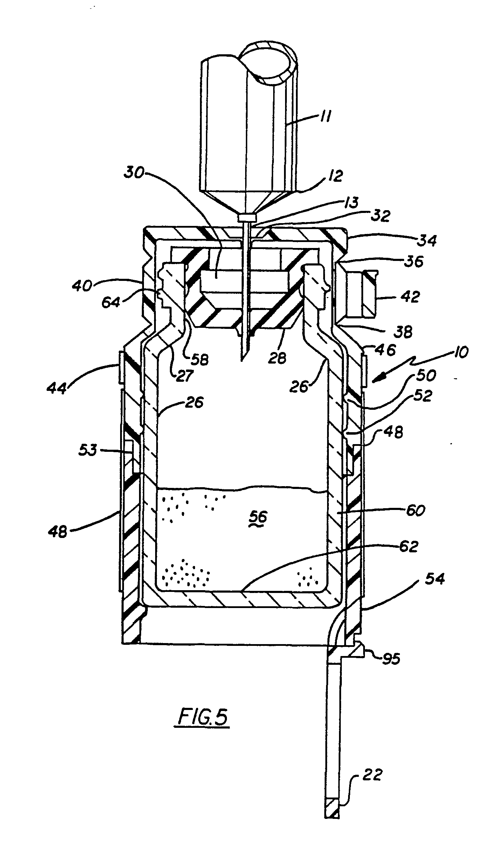

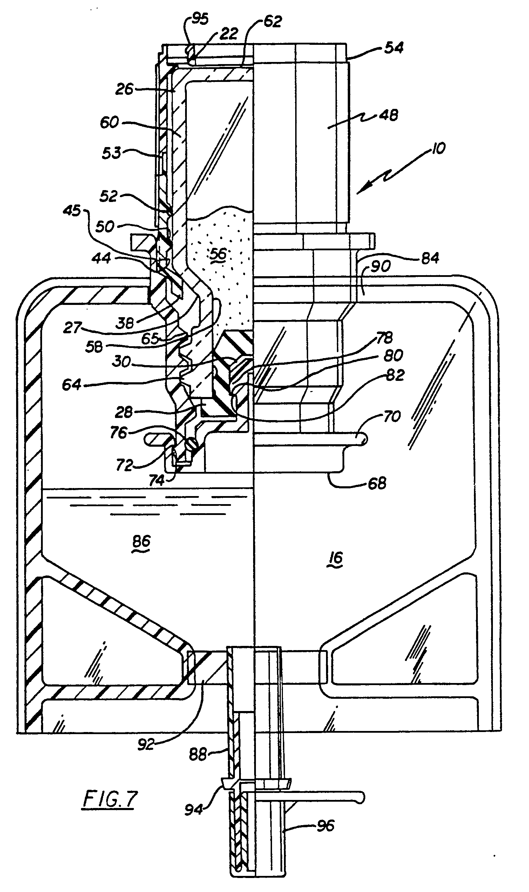

As will be explained in greater detail below, system 10 may also be used with a standard syringe 12 (FIGURE 5) once peelable seal 66 has been removed. In FIGURE 2, system 10 is shown forming part of a fluid source 16, typically a flexible container, which is further connected to tubing 18 and catheter or cannula 20 for intravenous administration of medicament and diluent to a patient. In this mode of operation the need to maintain the top portion of vial 26 and stopper 28 in a sterile condition can best be seen as neck 27 of vial 26 is in close proximity to the diluent in flexible container 16 (FIGURE 7).

The construction of system 10 which facilitates the maintenance of sterility and ease of operation is shown more specifically in FIGURES 3 and 4. A vial 26 is used to contain a flowable substance such as a powdered or liquid medicament 56. While a circular vial 26 having a tapered neck 27 is shown it will be understood that the shape of the vial is not critical to the operation of the system of the present invention.

A removable, pierceable stopper 28 seals opening 58 of vial 26 and maintains sterility of medicament 56. Stopper 28 is shown as being substantially U-shaped in the preferred embodiment; that is, having a depression 30 oriented so that the bottom of the U-shape is toward the medicament 56 and the arms of the U-shape seal against opening 58. It is to be understood that any stopper design such as one including a protuberance emanating outwardly from the stopper which facilitates removal of the stopper may be used in place of the U-shaped configuration shown.

The finish or top of vial 26 is shown in the preferred embodiment with threads 64 circumscribing the outside portion of opening 58. Alternatively a non-threaded vial 26 or vial whose finish includes an annular ring may be used.

Positioned over stopper 28 is a removable, pierceable diaphragm 32. Depending downward from the sides of pierceable diaphragm 32 is connection section 34. Connection section 34 terminates at frangible section 36 which circumscribes connection section 34. While a frangible section 36 is shown in connection with the preferred embodiment its presence is not critical to the operability of the invention. Alternatively any type of weakened section may be used. If desired, a second frangible section 38 may be used to form tear strip 40 which circumscribes the connection section 34 and the neck 27 of vial 26.

Further depending from tear strip 40 along the wall portion 60 of vial 26 is skirt member 46 which like connection section 34 circumscribes vial 26. On the inside of skirt member 46 are found rib members 50 and 52 which are in frictional engagement with wall portion 60 of vial 26. While rib member 50 and 52 are shown in connection with the preferred embodiment of the system 10 their presence is not critical to the operability of the invention. Alternatively, skirt member 46 may be in direct contact with either neck 27 or wall portion 60 of vial 26. In the preferred embodiment rib members 50 and 52 form a barrier to maintain neck 27, threads 64 and stopper 28 in a sterile condition. Therefore, pierceable diaphragm 32 is held in position over stopper 28 by being integral with connection section 34 which is in turn integral with skirt member 46 which is in frictional engagement with wall portion 60 of vial 26.

The outer surface of pierceable diaphragm 32 is protected and maintained in a sterile condition by peelable adhesive seal 66. Circumscribing skirt member 46 is a ring of ratchet teeth 44 which prevents removal of system 10 from a fluid source 16 (FIGURES 2 and 7). While ratchet teeth 44 are shown in connection with the preferred embodiment, their presence is not critical to the operability of the invention.

The bottom 62 and lower section of wall portion 60 of vial 26 are protected by shroud member 54 which is constructed to mechanically engage skirt member 46. While shroud member 54 is shown in conjunction with the preferred embodiment of system 10 its presence is not critical to the operability of the invention. Additionally, while stepped engagement 53 is shown between shroud member 54 and skirt member 46 any suitable method of fitting skirt member 46 and shroud member 54 together, such as a tongue and groove, may be used. Shroud member 54 has an integral swing-up hanger portion 22 which may be utilized to hang the system 10, such as shown in FIGURE 2. While hanger 22 is shown as a ring, any suitable design may be employed. Latch hook 98 may be added to hanger 22 in order to facilitate the hanging of system 10 as shown in FIGURE 2. Latch hook 98 engages the bottom of shroud member 54 when hanger 22 is swung away from bottom 62 of vial 26 as shown in phantom in FIGURE 4.

Label 48 may be utilized to maintain shroud member 54 in mechanical engagement 53 with skirt member 46 by placing that side of label 48 having adhesive in contact with skirt member 46 and shroud member 54. Alternatively frictional fitment such as threadable engagement or adhesive at engagement 53 may be used to maintain shroud member 54 in contact with skirt member 46.

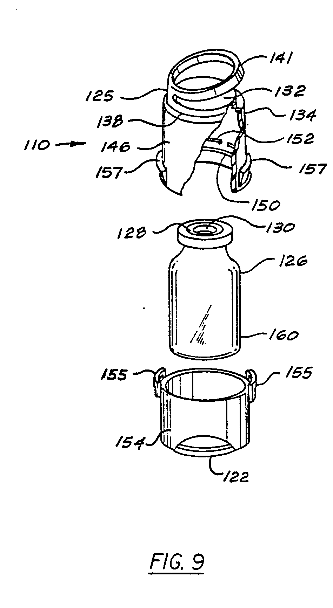

In the alternate embodiment 110 of the drug delivery system of the present invention, as shown in FIGURE 9, reference numbers in the "100" series have been used to designate those portions having similar construction, function and location to the parts of the preferred embodiment.

In the alternate embodiment 110, a pull ring 141 is used to apply mechanical force for the separation of pierceable diaphragm 132 from connection section 134 along tear detail 138. Hook means 155 engage ledges 157 on skirt member 146 to hold shroud member 154 in mechanical engagement with skirt member 146 and also in position over wall portion 160 of vial 126.

Operation

Utilization of system 10 with a syringe 12 is shown in FIGURE 5. This is accomplished by removing peelable adhesive seal 66 (FIGURE 4) and inserting cannula 13 through pierceable diaphragm 32. Once having passed through pierceable diaphragm 32, cannula 13 will then pass through depression 30 in stopper 28 before passing through the bottom portion of stopper 28 and entering the interior of vial 26 which contains medicament 56. If medicament 56 is a powder, diluent contained in barrel 11 of syringe 12 may be added through cannula 13 to the interior of vial 26, thereby allowing extraction of medicament 56 into barrel 11 of syringe 12 once medicament 56 has been dissolved in the diluent. If medicament 56 is in liquid form, it may be drawn directly from the interior of vial 26 into the barrel 11 of syringe 12.

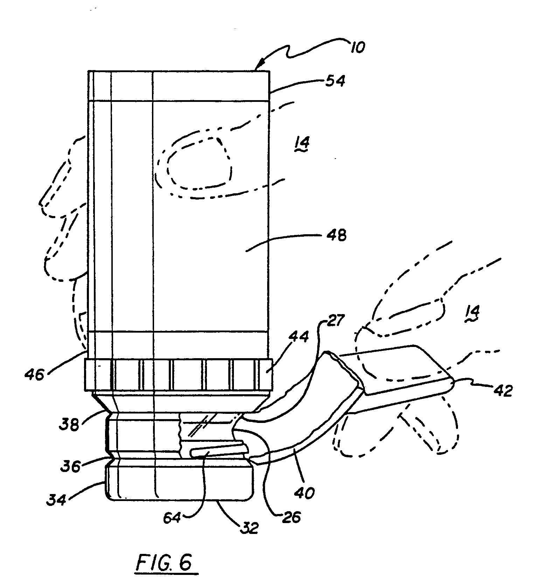

In the preferred mode of operation of system 10, as shown in FIGURES 6, 7 and 8, tab member 42 which is formed as part of tear strip 40 is grasped by fingers 14 and pulled away from skirt member 46. This pulling action will cause tear strip 40 to peel away from neck 27 of vial 26, by severing itself from connecting portion 34 and skirt member 46 at frangible sections 36 and 38. Once tear strip 40 has been completely removed from skirt member 46 and connection section 34, nothing remains to retain pierceable diaphragm 32 in its position over stopper 28 and it may therefore be removed from system 10.

As shown in FIGURE 7 neck 27 of vial 26 may now be inserted into port or sleeve 84 in fluid source 16 which is typically a flexible bag partially filled with diluent. This interengagement of vial 26 and sleeve 84 is accomplished by threadable engagement of threads 64 with complementary threads 65 within sleeve 84. Rotating vial 26 with respect to fluid source 16 causes neck 27 to be drawn into sleeve 84. This drawing action causes protuberance 78 from cover 68 on sleeve 84 to enter depression 30 in stopper 28. When protuberance 78 has completely entered depression 30 lip 80 of protuberance 78 will be in a position over ledge 82 in stopper 28. Ratchet teeth 44 engage compatible ratchet teeth 45 in sleeve 84. The slopes of compatible ratchet teeth 44 and 45 are such that system 10 cannot be backed out of sleeve 84 once interengagement has begun. Cover 68 is in sealing engagement with the bottom of sleeve 84 by the compression of O-ring 76 and by mechanical engagement of lip 72 over ledge 74 on the bottom of sleeve 84.

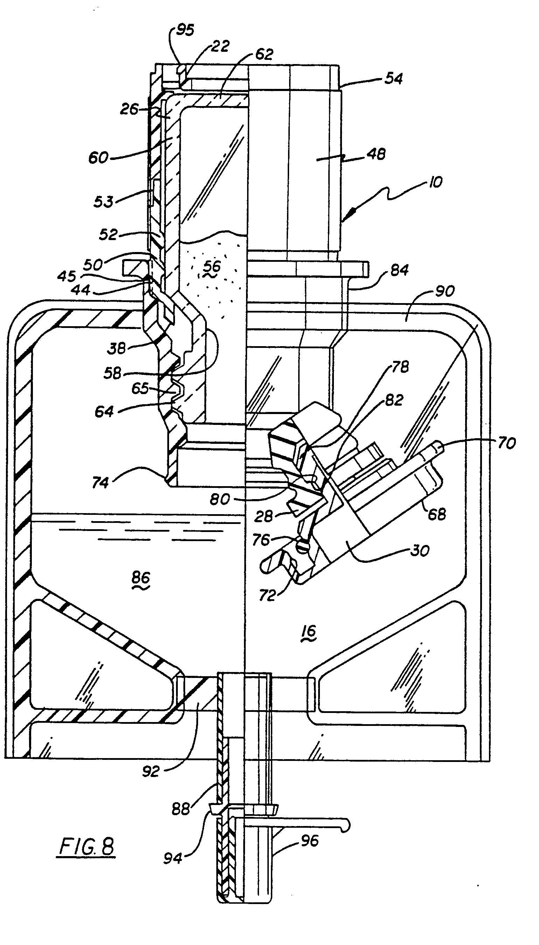

Once system 10 is in place in fluid source 16, medicament 56 may be mixed with diluent 86 as shown in FIGURE 8. By using the flexible properties of liquid source 16 the user may grasp flange 70 of cover 68. In doing so, the user may then manipulate cover 68 so that lip 72 rides over ledge 74, thus removing cover 68 from its mechanical engagement with the bottom of sleeve 84. As cover 68 is removed from sleeve 84, the mechanical engagement of lip 80 on protuberance 78 against ledge 82 on stopper 28 will cause stopper 28 to be removed along with cover 68. This manipulation of cover 68 — stopper 28 combination will create an open path through vial opening 58 for medicament 56 to intermix with diluent 86. Diluent 86 and medicament 56 may be further intermixed by squeezing the sides of liquid source 16. Hanger 22 may then be swung up and away from bottom 62 of vial 26 and used to hang the fluid source from a hook 24 on an I.V. pole 23 as shown in FIGURE 2. Hanger 22 is retained in position by the action of latch hook 98 at the base of hanger 22 which engages the bottom portion of shroud member 54. Tubing 18 and catheter or cannula 20 (FIGURE 2) are placed in fluid communication with the interior of fluid source 16 once cap 96 has been removed from port insert 94.

Should it be desired to administer two drugs simultaneously, a second drug may be added to vial 26 by use of syringe 12 before tear strip 40 is removed to utilize system 10 with fluid source 16. In the situation where a custom blend of medication is required, empty vials may be supplied and then later filled with the custom blend of medication for temporary storage before later use with a fluid source or a flexible fluid storage container.

Alternative embodiment 110 is operated in essentially the same manner as preferred embodiment 10; however pierceable diaphragm 132 is separated from skirt member 146 by means of a pull ring 141 rather than a tear strip 40 (FIGURE 6). Additionally vial 126 is shown with an unthreaded finish when utilization without threadable engagement such as a snap or frictional fitment is desired.

Systems 10 and 110 are made in substantially the same manner. Medicament 56 is first inserted into vial 26. Stopper 28 is then placed in opening 58 of vial 26. Skirt member 46 is then placed over vial 26 so that pierceable diaphragm 32 is positioned over stopper 28 and seal rings 50 and 52 are in frictional engagement with wall portion 60 of vial 26. Once in proper position, tear strip 40 will circumscribe neck 27 of vial 26. Shroud member 54 is then placed over the bottom of wall portion 60 and further moved to mechanically engage skirt member 46, as at stepped engagement 53. Label 48 is then wrapped around skirt member 46 and shroud member 54 so that skirt member 46 and shroud member 54 are maintained in close proximity.

Referring specifically to FIGURES 7 and 8, sleeve 84 is typically mandrel sealed 90 into the edge of liquid source 16 as is administration port 88 mandrel sealed 92 into another portion of liquid source or flexible container 16. Skirt member 46, tear strip 40, connecting portion 34, and pierceable diaphragm 32 of system 10 are typically made of a polypropylene plastic, as is shroud member 54. Alternatively, other suitable medical grade plastics such as polyvinyl chloride or polyethylene may be used. Vial 26 is constructed of glass; however it may also be constructed from polyethylene, polypropylene or any suitable medical grade barrier plastic or plastic combination. Stopper 28 is typically made of rubber; however, other suitable flexible medical grade plastics such as a styrene butadiene copolymer may be used in the place of rubber. Peelable adhesive seal 66 and label 48 are fabricated from paper or foil which has been coated with an adhesive compatible with the materials from which drug delivery system 10 has been fabricated. It should be further understood that the term "flowable" as employed in the specification and claims is meant to imply any material which will flow from one container to another whether liquid, solid or gas.

It will thus be seen that through the present invention there is now afforded a drug delivery system in which the procedures between storage and administration of a drug are minimized. Activation of the drug delivery system by health care or pharmacy personnel may be readily accomplished without the use of specially designed components or sophisticated methods which require an excessive number of procedures or prolonged exposure which might jeopardize sterility.

The foregoing invention can now be practised by those skilled in the art. Such skilled persons will know that the drug delivery system of the present invention is not necessarily restricted to the particular embodiments presented herein. The scope of the present invention is to be defined by the terms of the following claims as given meaning by the preceding description.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. A storage and delivery system for a flowable substance including a vial (26) having an opening (58), a wall portion (60), and a bottom (62), a removable stopper (28) in sealing engagement with said opening (58), and a diaphragm (32) positioned over said stopper (28), said system being characterized in that said stopper (28) and said diaphragm (32) are both pierceable, a skirt member (46) is in frictional engagement with said wall portion (60) of said vial (26), and weakened means (34) connect said diaphragm (32) to said skirt member (46) whereby said diaphragm (32) is also removable from its position over said stopper (28) upon breaking or removal of said weakened means (34).

2. The system according to claim 1 characterized in that said vial opening (58), said removable, pierceable stopper (28) and said wall portion (60) of said vial (26) enclosed by said means for connecting said removable, pierceable diaphragm (32) to said skirt member (46) are maintained in a sterile condition.

3. The system according to claim 1 or 2 characterized in that said means for connecting said removable, pierceable diaphragm (32) to said skirt member (46) is circumscribed by at least one frangible section (36).

4. The system according to claim 1, 2, or 3 characterized in that said removable, pierceable diaphragm (32) is maintained in a sterile condition by being covered with a peelable adhesive seal (66).

5. The system according to claim 1, 2, or 3, characterized in that frictional engagement of said skirt member (46) with the wall portion (60) of said vial (26) is provided by at least one rib member (50).

6. The system according to one or more of the preceding claims, characterized in that said weakened means (34) is circumscribed by a frangible portion (36) constructed and arranged to attach said removable, pierceable diaphragm (32) to said skirt member (46), and in that a shroud member (54) covers that section of said wall portion (60) of said vial (26) not covered by said skirt member (46), and wherein means (22) for hanging said storage and delivery system is formed as a part of said shroud member (54).

7. The system according to claim 1, 2, 3, 5 or 6 characterized in that it comprises in combination therewith a flexible fluid container (16) having a port (84), and in that said skirt member (46) and said port (84) in said flexible container (16) have complementary ratchet teeth (44, 45).

8. The system according to claim 1 or 2, characterized in that said stopper (28) has a depression (30) constructed and arranged to be engageable with means to remove said stopper from said vial (26).

9. The system according to claim 1, 2, 5, 6 or 8 in combination with a fluid container (16) having a port (84) and vial engagement means, characterized in that said vial (26) has complementary engagement means whereby fluid communication between the interior of said vial (26) and the interior of said fluid container (16) is accomplished by interengagement of said vial (26) with said port (84) in said fluid container (16).

10 The system according to claim 1, 2, 5, 6, 8 or 9 in combination with a fluid container (16) having a port (84) and threadable means (65) for engaging a vial (26) in association with said port (84), characterized in that said vial (26) has complementary threadable means (64) whereby interengagement of said opening (58) in said vial (26) with said port (84) in said fluid container (16) is accomplished by said complementary threadable means (64, 65).

11. The system according to claim 1, 2 or 8, characterized in that it further comprises a cannula (18, 20) inserted into said vial ·(26) through said diaphragm (32) and through said stopper (28), whereby said flowable substance may be extracted from said vial (26).

12. The system according to claim 1, 3 or 6, characterized in that said means (34) connecting said diaphragm (32) to said skirt member (46) is circumscribed by two frangible sections (36, 38) having a tab (42) located therebetween.

13. The system according to claim 6, characterized in that said shroud member (54) and said skirt member (46) are connected by a label (48) having an adhesive on one side, said adhesive being in contact with said shroud member (54) and skirt member (46).

14. The system according to claim 9, characterized in that said interengagement of said vial (26) with said port (84) in said fluid container (16) is accomplished by relative rotational engagement between said engagement means (64) and said complementary engagement means (65), and in that said system preferably further comprises complementary ratchet means (44) on said port (84) and said vial (26) for interengagement upon said relative rotational engagement whereby disengagement of said vial (26) from said fluid container (16) is prevented.

15. The system according to claims 1 and 14, characterized in that said ratchet means (44) on said vial (26) are defined by protuberances on said skirt member (46).

16. The system according to claim 1 or 2 and 7, 8, 9, 10, 11 or 14, characterized in that it further comprises means (70, 78, 80, 82) for removing said stopper (28) after said vial (26) is engaged with said fluid container (16), and preferably including means (82) provided on said stopper (28) which are adapted for engagement with stopper removing means (80) provided on said fluid container (16).

17. The system according to one or more of the preceding claims, characterized in that said complementary threadable means (65) comprise screw threads (65) adjacent said opening (58), and in that said ratchet means (44, 45) preferably comprise ratchet teeth formed on said port (84) and said vial (26).

18. The system according to claim 6, characterized in that said means (22) for hanging said storage and delivery system comprise a hanger (22) and means (98) to retain said hanger (22) in an outwardly disposed position relative to said vial (26) for facilitating the hanging of said system.

**Patentansprüche**

1. Aufbewahrungs- und Abgabesystem für eine fließfähige Substanz, mit einem Fläschchen (26), das eine Öffnung (58), einen Wandteil (60) und einen Boden (62) aufweist, einem entfernbaren Verschlußstopfen (28) in dichtendem Eingriff mit der Öffnung (58) und einer Membrane (32), die über dem Verschlußstopfen (28) angeordnet ist, wobei das System dadurch gekennzeichnet ist, daß sowohl der Verschlußstopfen (28) als auch die Membrane (32) perforierbar sind, ein Mantelelement (46) in Reibungseingriff mit dem Wandteil (60) des Fläschchens (26) steht und Schwächungsmittel (34) die Membrane (32) mit dem Wandelement (46) verbinden, so daß die Membrane (32) aus ihrer Position über dem Verschlußstopfen (28) beim Brechen oder Entfernen der Schwächungsmittel (34) ebenfalls entfernbar ist.

2. System nach Anspruch 1, dadurch gekennzeichnet, daß die Fläschchenöffnung (58), der entfernbare perforierbare Verschlußstopfen (28) und der Wandteil (60) des Fläschchens (26), welcher von den Mitteln zum Verbinden der entfernbaren perforierbaren Membrane (32) mit dem Mantelelement (46) umschlossen ist, in einem sterilen Zustand gehalten werden.

3. System nach Anspruch 1 oder 2, dadurch gekennzeichnet, daß die Mittel zum Verbinden der entfernbaren perforierbaren Membrane (32) mit dem Mantelelement (46) von zumindest einem zerbrechlichen Abschnitt (36) begrenzt sind.

4. System nach einem der Ansprüche 1 bis 3, dadurch gekennzeichnet, daß die entfernbare perforierbare Membrane (32) in einem sterilen Zustand gehalten wird, indem sie mit einer abschälbaren Klebedichtung (66) bedeckt ist.

5. System nach Anspruch 1, 2 oder 3, dadurch gekennzeichnet, daß der Reibungseingriff des Mantelelementes (46) mit dem Wandteil (60) des Fläschchens (26) durch zumindest ein Rippenelement (50) gebildet wird.

6. System nach einem oder mehreren der vorhergehenden Ansprüche, dadurch gekennzeichnet, daß die Schwächungsmittel (34) von einem zerbrechlichen Teil (36) begrenzt sind, der so ausgebildet und angeordnet ist, daß er die entfernbare perforierbare Membrane (32) an dem Mantelelement (46) befestigt, und daß ein Abdeckelement (54) jenen Abschnitt des Wandteiles (60) des Fläschchens (26) abdeckt, der vom Mantelelement (46) nicht bedeckt ist, wobei Mittel (22) zum Aufhängen des Aufbewahrungs- und Abgabesystems als Teil des Abdeckelementes (54) ausgebildet sind.

7. System nach einem der Ansprüche 1, 2, 3, 5 oder 6, dadurch gekennzeichnet, daß es mit einem flexiblen Fluidbehälter (16) kombiniert ist, der eine Öffnung (84) hat, und daß das Mantelelement (46) und die Öffnung (84) im flexiblen Behälter (16) komplementäre Sperrzähne (44, 45) aufweisen.

8. System nach Anspruch 1 oder 2, dadurch gekennzeichnet, daß der Verschlußstopfen (28) eine Vertiefung (30) aufweist, die so ausgebildet und angeordnet ist, daß Mittel zum Entfernen des Verschlußstopfens von dem Fläschchen (26) mit ihr in Eingriff versetzbar sind.

9. System nach einem der Ansprüche 1, 2, 5, 6 oder 8 in Kombination mit einem Fluidbehälter (16), der eine Öffnung (84) und Eingriffsmittel für das Fläschchen aufweist, dadurch gekennzeichnet, daß das Fläschchen (26) komplementäre Eingriffsmittel aufweist, so daß eine Fluidverbindung zwischen dem Inneren des Fläschchens (26) und dem Inneren des Fluidbehälters (16) durch gegenseitigen Eingriff des Fläschchens (26) und der Öffnung (84) im Fluidbehälter (16) erzielt wird.

10. System nach einem der Ansprüche 1, 2, 5, 6, 8 oder 9 in Kombination mit einem Fluidbehälter (16), der eine Öffnung (84) und Gewindemittel (65) zum Angriff an einem Fläschchen (26) in Verbindung mit der Öffnung (84) aufweist, dadurch gekennzeichnet, daß das Fläschchen (26) komplementäre Gewindemittel (64) aufweist, so daß ein gegenseitiger Eingriff der Öffnung (58) im Fläschchen (26) und der Öffnung (84) im Fluidbehälter (16) durch die komplementären Gewindemittel (64, 65) erreicht wird.

11. System nach einem der Ansprüche 1, 2 oder 8, dadurch gekennzeichnet, daß es ferner eine Kanüle (18, 20) aufweist, die in das Fläschchen (26) durch die Membrane (32) und durch den Verschlußstopfen (28) hindurch eingeführt wird, so daß die fließfähige Substanz aus dem Fläschchen (26) extrahiert werden kann.

12. System nach einem der Ansprüche 1, 3 oder 6, dadurch gekennzeichnet, daß die Mittel (34), welche die Membrane (32) mit dem Mantelelement (46) verbinden, von zwei zerbrechlichen Abschnitten (36, 38) begrenzt sind, die eine dazwischen angeordnete Zunge (42) aufweisen.

13. System nach Anspruch 6, dadurch gekennzeichnet, daß das Abdeckelement (54) und das Mantelelement (46) durch ein Klebeettikett (48) verbunden sind, das auf einer Seite Klebstoff aufweist, wobei der Klebstoff in Kontakt mit dem Abdeckelement (54) und dem Mantelelement (46) ist.

14. System nach Anspruch 9, dadurch gekennzeichnet, daß der gegenseitige Eingriff des Fläschchens (26) und der Öffnung (84) im Fluidbehälter (16) durch relativen Dreheingriff zwischen den Eingriffsmitteln (64) und den komplementären Eingriffsmitteln (65) erreicht wird, und daß das System vorzugsweise ferner komplementäre Sperrmittel (44) an der Öffnung (84) und dem Fläschchen (26) zum gegenseitigen Eingriff bei dem relativen Dreheingriff aufweisen, wobei ein Lösen des Fläschchens (26) vom Fluidbehälter (16) verhindert wird.

15. System nach den Ansprüchen 1 und 14, dadurch gekennzeichnet, daß die Sperrmittel (44) am Fläschchen (26) durch Vorsprünge am Mantelelement (46) gebildet sind.

16. System nach einem der Ansprüche 1 oder 2 und 7, 8, 9, 10, 11 oder 14, dadurch gekennzeichnet, daß es ferner Mittel (70, 78, 80, 82) aufweist, um den Verschlußstopfen (28) zu entfernen, nachdem das Fläschchen (26) mit dem Fluidbehälter (16) in Eingriff gebracht worden ist, und vorzugsweise Mittel (82) aufweist, die am Verschlußstopfen (28) vorgesehen und so ausgebildet sind, daß sie mit den Verschlußstopfenentfernungsmitteln (80) in Eingriff kommen, die am Fluidbehälter (16) vorgesehen sind.

17. System nach einem oder mehreren der vorhergehenden Ansprüche, dadurch gekennzeichnet, daß die komplementären Gewindemittel (65) Schraubgewinde (65) nahe der Öffnung (58) aufweisen und daß die Sperrmittel (44, 45) vorzugsweise Sperrzähne aufweisen, die an der Öffnung (84) und am Fläschchen (26) ausgebildet sind.

18. System nach Anspruch 6, dadurch gekennzeichnet, daß die Mittel (22) zum Aufhängen des Aufbewahrungs- und Abgabesystems einen Hänger (22) und Mittel (98) aufweisen, um den Hänger (22) in auskragender Stellung relativ zum Fläschchen (26) zu halten, um das Aufhängen des Systems zu erleichtern.

## Revendications

1. Ensemble de stockage et de distribution d'une substance fluide, comprenant un flacon (26) ayant une ouverture (58), une partie de paroi (60) et un fond (62), un bouchon amovible (28) coopérant de façon étanche avec l'ouverture (58), et un diaphragme (32) placé sur le bouchon (28), l'ensemble étant caractérisé en ce que le bouchon (28) et le diaphragme (32) peuvent tous deux être percés, une jupe (46) coopère par frottement avec la partie de paroi (60) du flacon (26), et un dispositif de résistance réduite (34) raccorde le diaphragme (32) à la jupe (46), si bien que le diaphragme (32) peut aussi être retiré de son emplacement au-dessus du bouchon (28) après rupture ou enlèvement du dispositif de résistance réduite (34).

2. Ensemble selon la revendication 1, caractérisé en ce que l'ouverture (58) du flacon, le bouchon amovible (28) qui peut être percé et ladite partie de paroi (60) du flacon (26) qui est entourée par le dispositif de raccordement du diaphragme amovible (32), qui peut être percé, à la jupe (46) sont maintenus à l'état stérile.

3. Ensemble selon la revendication 1 ou 2, caractérisé en ce que le dispositif de raccordement du diaphragme amovible (32), qui peut être percé, à la jupe (46) est, entouré par au moins un tronçon cassable (36).

4. Ensemble selon la revendication 1, 2 ou 3, caractérisé en ce que le diaphragme amovible (32), qui peut être percé, est maintenu à l'état stérile par recouvrement par un joint adhésif pelable (66).

5. Ensemble selon la revendication 1, 2 ou 3, caractérisé en ce que la coopération par frottement de la jupe (46) et de la partie de paroi (60) du flacon (26) est assurée par au moins une nervure (50).

6. Ensemble selon l'une quelconque des revendications précédentes, caractérisé en ce que le dispositif de résistance réduite (34) est entouré par une partie cassable (36) ayant une construction et une disposition telles que le diaphragme amovible (32) et qui peut être percé est fixé à la jupe (46), et en ce qu'un organe protecteur (54) recouvre le tronçon de partie de paroi (60) du flacon (26) qui n'est pas recouvert par le jupe (46), et un dispositif (22) de suspension de l'ensemble de stockage et de distribution est formé par une partie de l'organe protecteur (54).

7. Ensemble selon la revendication 1, 2, 3, 5 ou 6, caractérisé en ce qu'il comporte, en combinaison avec lui, un récipient souple (16) de fluide ayant un canal (84), et la jupe (46) et le canal (84) du récipient souple (16) ont des dents complémentaires d'encliquetage (44, 45).

8. Ensemble selon la revendication 1 ou 2, caractérisé en ce que le bouchon (28) a une cavité (30) dont la construction et la disposition sont telles que la cavité peut coopérer avec un dispositif d'extraction du bouchon du flacon (26).

9. Ensemble selon la revendication 1, 2, 5, 6 ou 8 en combinaison avec un récipient (16) de fluide ayant un canal (84) et un dispositif de coopération avec un flacon, caractérisé en ce que le flacon (26) a un dispositif de coopération complémentaire, si bien que la communication permettant la circulation du fluide entre l'intérieur du flacon (26) et l'intérieur du récipient de fluide (16) est, réalisée par mise en coopération du flacon (26) avec le canal (84) du récipient de fluide (16).

10. Ensemble selon la revendication 1, 2, 5, 6, 8 ou 9, en combinaison avec un récipient de fluide (16) ayant un canal (84) et un dispositif (65) qui peut être vissé pour la mise en coopération d'un flacon (26) avec le canal (84), caractérisé en ce que le flacon (26) a un dispositif complémentaire (64) de coopération par vissage, si bien que la mise en coopération de l'ouverture (58) du flacon (26) avec le canal (84) du récipient de fluide (16) est réalisée à l'aide des dispositifs complémentaires (64, 65) de coopération par vissage.

11. Ensemble selon la revendication 1, 2 ou 8, caractérisé en ce qu'il comporte en outre une canule (18, 20) introduite dans le flacon (26) à travers le diaphragme (32) et le bouchon (28), si bien que la substance fluide peut être extraite du flacon (26).

12. Ensemble selon la revendication 1, 3, 3 ou 6, caractérisé en ce que le dispositif (34) raccordant le diaphragme (32) à la jupe (46) est entouré par deux tronçons cassables (36, 38) ayant une patte (42) qui est placée entre eux.

13. Ensemble selon la revendication 6, caractérisé en ce que l'organe protecteur (54) et la jupe (46) sont raccordés par une étiquette (48) ayant un adhésif d'un côté, l'adhésif étant au contact de l'organe protecteur (54) et de la jupe (46).

14. Ensemble selon la revendication 9, caractérisé en ce que la coopération du flacon (26) et du canal (84) du récipient de fluide (16) est assurée par mise en contact et rotation relative du dispositif de coopération (64) et du dispositif complémentaire de coopération (65), et en ce que l'ensemble comporte en outre de préférence des dispositifs complémentaires d'encliquetage (44) formés sur le canal (84) et sur le flacon (26) et destinés à coopérer lors du contact et de la rotation relative si bien que le flacon (26) ne peut pas être séparé du récipient de fluide (16).

15. Ensemble selon les revendications 1 et 14, caractérisé en ce que le dispositif d'encliquetage (44) formé sur le flacon (26) est délimité par des protubérances de la jupe (46).

16. Ensemble selon la revendication 1 ou 2 et 7, 8, 9, 10, 11 ou 14, caractérisé en ce qu'il comporte en outre un dispositif (70, 78, 80, 82) destiné à retirer le bouchon (28) après que le flacon (26) a été mis en coopération avec le récipient de fluide (16), et comprenant de préférence un dispositif (82) placé sur le bouchon (28) et destiné à coopérer avec le dispositif (80) d'extraction du bouchon placé sur le récipient de fluide (16).

17. Ensemble selon une ou plusieurs des revendications précédentes, caractérisé en ce que les dispositifs complémentaires (65) de coopération par vissage comportent des filets (65) placés près de l'ouverture (58), et en ce que les dispositifs d'encliquetage (44, 45) comprennent de préférence des dents d'encliquetage formées sur le canal (84) et le flacon (26).

18. Ensemble selon la revendication 6, caractérisé en ce que le dispositif (22) de suspension de l'ensemble de stockage et de distribution comporte un organe de suspension (22) et un dispositif (98) destiné à retenir l'organe de suspension (22) en position tournée vers l'extérieur par rapport au flacon (26) afin que la suspension de l'ensemble soit facilitée.

FIG.1

FIG.2

FIG.3

*FIG. 4*

*FIG.5*

10

54

14

48

46

44

27

38

14

42

36

40

34

64

32

26

FIG. 6

*FIG.7*

FIG.8

FIG. 9